(19)

Europäisches Patentamt
European Patent Office
Office européen des brevets

(11) **EP 4 278 974 A1**

(12) **EUROPEAN PATENT APPLICATION**

(43) Date of publication:
**22.11.2023 Bulletin 2023/47**

(21) Application number: **22173972.5**

(22) Date of filing: **18.05.2022**

(51) International Patent Classification (IPC):
*A61B 6/00* *(2006.01)*    *A61B 8/08* *(2006.01)*

(52) Cooperative Patent Classification (CPC):
**A61B 6/481; A61B 6/507; A61B 6/5217**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HAASE, Hans, Christian**
**Eindhoven (NL)**
• **GRASS, Michael**
**Eindhoven (NL)**
• **VAN LAVIEREN, Martijn, Anne**
**5656AG Eindhoven (NL)**

(74) Representative: **Philips Intellectual Property & Standards**
**High Tech Campus 52**
**5656 AG Eindhoven (NL)**

(54) **ESTIMATION OF CONTRAST INDUCED HYPEREMIA FROM SPECTRAL X-RAY IMAGING**

(57)    System (SYS) and related method for image processing. The system comprises an input interface (IN) for receiving input data including projection imagery of a patient with contrast agent acquired by an X-ray based imaging apparatus (IA). An analyzer (AZ) of the system (SYS) is configured to establish a patient state, including a hyperemic state or a resting state, based on the projection imagery. An output interface (OUT) provides an output signal indicative of the established state for facilitating obtaining a hemodynamic index in relation to the patient.

**FIG. 1**

**Description**

FIELD OF THE INVENTION

The invention relates to a system for supporting computation of a hemodynamic index, to a related method, to a system for training a machine learning model for use in such a system for supporting computation of the hemodynamic index, to a related method for training a machine learning model, to a medical imaging arrangement, to a computer program element, and to a computer readable medium.

BACKGROUND OF THE INVENTION

For an accurate diagnosis of patients presenting with symptoms of coronary artery disease, multiple hemodynamic indices have been established.

Some such indices are based on certain hemodynamic measurements that must be measured in a certain microvascular state, such as in a resting state [e.g. iFR (instantaneous wave-free ratio)], others in a hyperemic state [e.g. FFR (Fractional flow reserve], IMR (index of microvascular resistance)], and some combine measurements from both states [e.g. CFR (coronary flow reserve)].

An incorrect microvascular state for a measurement may be a significant source of error for diagnosis and may lead to wrong treatment.

Administration of drugs to induce hyperemia is an added burden to related invasive procedures, and may cause significant discomfort to the patient.

The administration of contrast agent, which is a routine element of some of such procedures, also causes short hyperemic states it has been found. This observation may provide an opportunity to avoid the use of adenosine or other hyperemia inducing medication. On the other hand, such contrast induced hyperemia may unknowingly effect measurements and may lead to false results.

SUMMARY OF THE INVENTION

There may therefore be a need for improving handling of hemodynamic indices in procedures relating to such indices.

An object of the present invention is achieved by the subject matter of the independent claims, wherein further embodiments are incorporated in the dependent claims. It should be noted that the following described aspect of the invention equally applies to the related method, to medial the imaging arrangement, to the system for training a machine learning model, to the method of training a machine learning model, to the computer program element and to the computer readable medium.

According to a first aspect of the invention there is provided a system for image processing, comprising:

an input interface for receiving input data including projection imagery of a patient with contrast agent acquired by an X-ray based imaging apparatus (IA);

an analyzer configured to establish a patient state, including a hyperemic state or a resting state, based on the projection imagery; and

an output interface for providing an output signal indicative of the established state for facilitating obtaining a hemodynamic index in relation to the patient.

In embodiments, the facilitator is operative to associate respective hemodynamic measurement with a respective patient state to obtain associated data.

In embodiments, the system comprises an index computation component, operable to compute one or more hemodynamic indices based on the associated data.

In embodiments, the system comprises a facilitator configured to facilitate collection of hemodynamic measurements based on the established patient state.

The hemodynamic measurements may be collected by in-situ sensors or may be collected based on image analysis.

In embodiments, the analyzer is to correlate contrast agent amount/concentration as per the received imagery with patient state.

In embodiments, the analyzer is based on a trained machine learning model.

In embodiments, the model includes an artificial neural network model.

A machine learning approach allows taking context data into account and allows for more robust learning of patient specific differences. However, such an ML based approach is not necessarily required herein in all embodiments.

Instead, analytic functional modelling, based on physical or physiological insight with explicit calculation may be used instead. For example. LUTs may be used in some embodiments to estimate the state.

In embodiments, the facilitator includes a graphics display generator/visualizer configured to effect display on a display device of an indication of the established state.

In embodiments, the system includes as facilitator configured to control acquisition of such hemodynamic measurements as a function of the established state. For example, the acquisition may be in synchrony or anti-synchrony, etc with the established state.

In embodiments, the system includes a measurement output data interface for providing one or more such hemodynamic measurements.

In embodiments, the measurement output data interface is to provide the one or more hemodynamic measurements for displaying on the, or a, display device. Alternatively, or in addition, the hemodynamic measurements are stored in a memory, are provided for medical purposes, or other otherwise utilized.

In embodiments, the input data further includes in-situ pressure or flow measurements and the analyzer is configured to establish the patient state, further based on the in-situ pressure or flow measurements.

In embodiments, the analyzer is to establish an over- time behavior of the patient state, the analyzer to use the same over-time behavior for establishing a next patient state of the patient with a non-contrast agent substance instead of the contrast agent, the non-contrast agent substance capable of inducing a patient state similar to the patient state induced by the contrast agent. No projection imagery is needed anymore in this case to estimate patient state. Saline solutions are typically used to flush or dilute. Its advantage is that it is not harmful to the patient as compared to contrast agents. However, saline solutions are merely one example, but other non-harmful, hyperemia inducing substances may be used instead.

In embodiments, the imaging apparatus is configured for spectral imaging. Spectral imaging may include acquiring multi-energy projection data and processing this data by a spectral imaging algorithm such as material decomposition, or other to obtain the spectral imagery. Spectral imagery may be in projection domain or in image domain.

In embodiments, the imaged region of interest includes at least a part of the heart of the patient or a peripheral vessel.

In embodiments, the facilitator uses additional 3D image input from e.g. CT, MR or other data. This allows achieving yet better robustness.

In another aspect there is provided a medical imaging arrangement, including the system of any one of the previous claims and i) the imaging apparatus.

In embodiments, the arrangement further includes any one of more of: i) the display device, ii) a contrast or non-contrast agent delivery apparatus, iii) device configured to acquire the hemodynamic measurements.

The measurement device may include a pressure wire, or another such as flow-measurement device, etc

In another aspect there is provided a training system configured to train the machine learning model based on training data.

In yet another aspect there is provided a computer-implemented method for image-processing, comprising:

receiving input data including projection imagery of a patient with contrast agent acquired by an X-ray based imaging apparatus;

establishing a patient state, including a hyperemic state or a resting state, based on the projection imagery; and providing an output signal indicative of the established state for facilitating obtaining a hemodynamic index in relation to the patient.

In embodiments, the method includes computing the hemodynamic index based on measurements associated with predefined types of hyperemic states. The established hyperemic states may be used to ensure that the associated hyperemic states are of the correct type as pre-defined. The said types may include any one or more of: fully hyperemic, rest state or a state intermediate between the two. The method may include using the established state to correct a previously calculated index that was based on measurements collected at a incorrect state.

The method may include synchronizing or coordinating collection of hemodynamic measurements. This may be done in a pre- or post-gating

The method may include controlling imaging related tasks based on the established patient state.

In another aspect there is provided a computer implemented method for training the machine learning model based on training data.

In another aspect there is provided a computer program element, which, when being executed by at least one processing unit, is adapted to cause the at least one processing unit to perform the method.

In another aspect there is provided at least one computer readable medium having stored thereon the program element, or having stored thereon the trained machine learning model.

The system or method may be used for facilitating computation of one or more hemodynamic indices.

The system or method may thus implement an explicit and distinct step to establish the hyperemic patient state. In addition, based on the established states, hemodynamic measurements may be collected.

In addition or instead, the established hyperemic state may be used to trigger or control imaging related tasks, or may otherwise support imaging.

The computed hyperemic state may be used for various control tasks. The system may help avoid mistakes in relation to hyperemic states, and at the same time still allows using contrast induced hyperemia as an advantage. With the proposed system, hemodynamic measurements may be performed under due consideration of contrast agent administration and its effect on hyperemia.

It is also proposed herein in some embodiments to use spectral X-ray imaging, for example in the cathlab, to monitor contrast injections, estimate the hyperemic state and possibly correct and/or validate hemodynamic measurements accordingly.

In combination with image derived physiology methods, the proposed system-can be used with benefit. Hemodynamic parameters that are extracted from angiography sequences are hereby directly linkable to hyperemic states of the patient. This improves automation and reduces user dependence, e.g. for image derived IMR, CFR, or extraction of iFR boundary conditions.

Thus, the proposed system and method may serve as an enabler for contrast- induced and image-derived IMR measurements with improved, if not optimal, ease of use.

However, the system and method may also be used with benefit in scenarios where external in-situ measurement equipment. such as pressure/flow wires, etc, are used.

What is proposed herein in embodiments is to use a spectral imaging apparatus, such as a C-arm X-ray imaging system equipped with a dual-layer detector or otherwise, that delivers spectral intraprocedural imagery. The proposed system is operable to calculate for example a material decomposition to extract therefrom information about the contrast agent (eg, Iodine) dose and/or concentration in vessels and/or tissue. The system relates the calculated contrast agent (eg Iodine) measurement, or the spectral imagery as a whole, to a hyperemic state of the patient. The system may combine the determined hyperemic state with diagnostic hemodynamic measurements, for example to display alerts/warnings, corrected values, enable specific measurements, etc.

The term *"user"* relates to a person, such as medical personnel or other, operating the imaging apparatus or overseeing the imaging procedure. In other words, the user is in general not the patient.

In general, the term *"machine learning"* includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. Training experience helps improve performance if the training data well represents a distribution of examples over which the final system performance is measured. The performance may be measured by objective tests based on output produced by the module in response to feeding the module with test data. The performance may be defined in terms of a certain error rate to be achieved for the given test data. For more details, see for example, T. M. Mitchell, "Machine Learning", page 2, section 1.1, page 6 1.2.1, McGraw-Hill, 1997.

A *"hemodynamic index"* is a measure, such as a scalar value (number) or a group of numbers that described dynamic behavior of blood in a region of interest. The index may serve as a standardized measure for diagnosis and treatment decisions.

BRIEF DESCRIPTION OF THE DRAWINGS

Exemplary embodiments of the invention will now be described with reference to the following drawings, which, unless stated otherwise, are not to scale, wherein:

Figure 1 shows a block diagram of a medical imaging arrangement;
Figure 2 shows as block diagram of a system for facilitating computing a hemodynamic index;
Figure 3 shows a more detailed block diagram of the system of Figure 2;
Figure 4 shows a bock diagram of a machine learning model;
Figure 5 show a block diagram of a training system for training a machine learning model;
Figure 6 shows a flow chart of a computer-implemented method for facilitating computing a hemodynamic index; and
Figure 7 shows a flow chart of a computer-implemented method for training a machine learning model.

DETAILED DESCRIPTION OF EMBODIMENTS

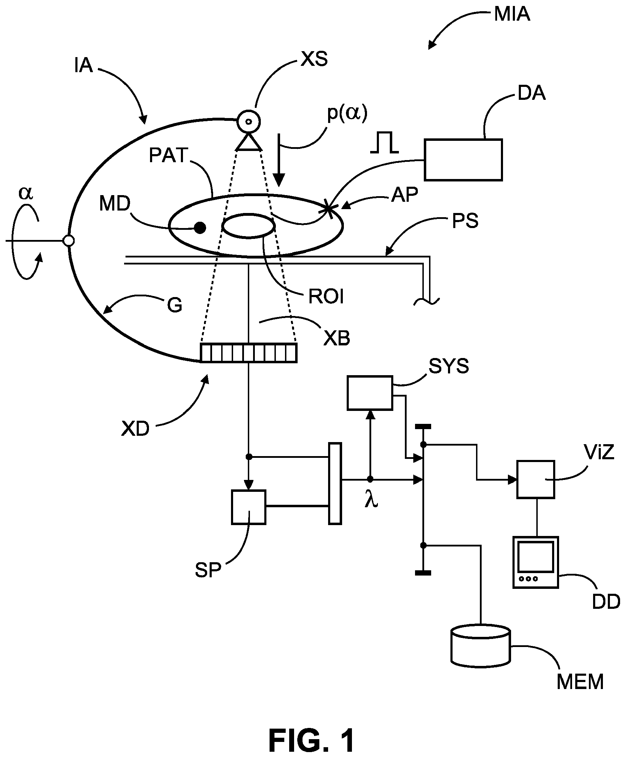

Figure 1 provides a block diagram of a medical imaging arrangement MIA configured to facilitate computing certain hemodynamic indices which may be used to assess a blood vessel lesion in a patient.

Main applications include herein cardiac examinations where a stricture in a coronary vessel of the patient PAT's heart needs to be assessed based on those one or more hemodynamic indices. Other applications may also be envisaged herein. Such a hemodynamic index may be computed based on hemodynamic quantities collected at or around the lesion. Such hemodynamic quantities may include flow, volume or pressure measurements, or other one or more quantities that are suitable to describe the dynamic behavior of, in particular, blood and/or other fluids at or around the lesion. It has been observed that the accuracy of such indices can be markedly improved when the associated measurements are collected under correct conditions. Such a condition may include a hyperemic state of the region of interest ROI. The ROI may include a neighborhood around the lesion, such as around the said arterial or veinal stricture. Thus, in general, as understood herein the region of interest includes the lesion, such as the stricture, or a neighborhood around the lesion. Further diagnostic or even therapeutic measures may be informed by knowledge of the hemodynamic indices.

As envisaged herein, a computer-implemented system SYS facilitates, preferably image-based, collection of the hemodynamic measurement(s) during the respective correct hyperemic state. The correct hyperemic is pre-associated with the respective type of hemodynamic index one wishes to compute. Operation of system SYS will be explained below in more detail, after first describing the medical imaging arrangement MIA more broadly.

The medical imaging arrangement MIA may include a, preferably, X-ray based imaging apparatus IA (also referred to herein simply as "imager"). The imaging apparatus IA may be of the rotational type. Such a rotational imaging apparatus allows collection of projection imagery $\lambda$ along different projection directions $P(\alpha)$ around the region of interest, such as around the mentioned stricture in a coronary vessel. The imaging apparatus IA includes an X-ray source XS and an X-ray sensitive detector XD. In some embodiments, the X-ray source XS, and opposite thereto, the X-ray sensitive detector XD, are arranged preferably on a rotatable gantry G. The gantry G may have a recess in which an examination region is defined where the patient, or in particular the region of interest, resides during imaging. The rotatable gantry may thus have a "C", "U" or similar shape, such as in C-arm imaging apparatus envisaged herein. However, such or similar rotational or an otherwise dynamic setup, where there is at least some relative motion between source and detector intended, is not necessarily required herein in all embodiments. Instead, a planar radiographic setup is also envisaged in some embodiments. In addition, whilst digital-at-source imaging, such as flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc with post-digitization is also envisaged in some embodiments.

Generally, imaging includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary vessels, provide usually poor contrast in native X-ray imagery. In order to boost contrast, a contrast agent (sometimes "CA" or short), such as an iodine solution, or other, is delivered through an access point AP into the blood flow of the patient prior to or during imaging. The contrast agent CA may be delivered via a CA delivery apparatus DA, such as a motorized pump, but manual delivery is not excluded herein. The delivery apparatus DA may be operable to deliver a defined volume of contrast agent to the patient. The volume of contrast agent so delivered travels with the blood flow. After a certain period of time contrast agent accumulates at the region of interest such as in or around the stricture. Image acquisition is preferably synchronized with arrival of contrast agent at the ROI. Preferably "contrasted" imagery is acquired whilst there is contrast agent CA present at the ROI. Optionally, additional "non-contrasted" imagery is acquired whilst no contrast agent is present at the ROI.

The in particular contrasted imagery may be acquired in a time series with varying concentration of the contrast agent accumulating at the region of interest. Image acquisition may start before, may continue throughout, and may terminate after accumulation of the contrast agent at the ROI. In general, contrast agent concentration increases over time after delivery, will plateau at a certain maximum value, and will then wash out or decrease over time until completely vanished. Whilst a single or certain discrete number of still images may be acquired in some instances, acquisition of a stream images, or frames, acquired at a suitable frame rate is preferred as this allows real time dynamic observation. Specifically, the acquired stream of projection imagery may be visualized by a visualizer VIZ as a live video feed on display device DD. Alternatively or in addition, still imagery is so visualized. Operation of the facilitator system SYS is preferably based on processing such still or stream imagery. The imagery may be stored in an image memory MEM or may be processed otherwise.

X-ray imaging is generally based on the attenuation co-efficient of the matter(s) that makes up the region of interest. The attenuation (coefficient) is dependent on the energy of the X-ray beam. In order to harness this energy dependency, a spectral imager IA may be used, configured for spectral imaging. This may include detector-sided or source-sided solutions. Detector-sided solutions include photon-counting detector hardware or a dual-energy setup with dual-layer detector hardware for example. More than two detector layers may be used although this is less common. Source-side solutions include multi-source setups, kVp switching, filtration and other.

The imaging set-up in spectral imaging allows acquisition of multi-dimensional image data. Such multi-dimensional image data includes projection imagery at different energy levels, instead of acquiring energy integrated imagery as provided by a conventional (non-spectral) X-ray imaging set-up, the latter not being excluded herein. For example, such a conventional set-up may be used for digital angiography subtraction ("DAS") where pairs of projection images at higher and lower concentration of the contrast agent is acquired. Such image pairs are then spatially registered and subtracted from each other to boost contrast of the soft tissue including the contrast agent. DSA may also be practiced with spectral imaging setup.

Preferably however, for more accurate quantification of the contrast agent concentration/volume at the region of interest, a spectral set-up is preferred. Such a spectral imaging setup includes a spectral image processor SP. The spectral image processor SP may implement a spectral image processing algorithm, such as material decomposition or other. A range of such spectral image processing algorithms are envisaged herein. The spectral processor SP processes the multi-energy projection imagery $\lambda$ acquired by the source- or detector-sided imaging set-up just described, and computes therefrom spectral imagery that differs from the original input multi-energy projection imagery. Spectral imagery is thus computationally derived from the input multi-energy projection imagery. Such spectral imagery, which may be in projection domain, may include for example, a monochromatic image at a desired energy level, or a virtual non-contrast image, or a contrast-only image (e.g. iodine image), a Compton scatter image, and others. The monochromatic image may correspond to the attenuation co-efficient of the contrast agent used. For example, the energy value or energy range for the monochromatic image may be chosen to equal or include the K-edge energy of the particular contrast agent to be used for good contrast for example. However, such as K-edge based choice is not mandatory herein and other energy values or ranges for the mono-chromatic imagery is equally envisaged herein. The monochromatic image represents a computational approximation of an image that may have been obtained had a monochromatic X-ray source XS at the respective energy level been used for imaging. In most practical cases, the actual X-ray source XS is polychromatic. The virtual non contrast ("VNC") image represents an approximation of an image that may have been obtained if there had been no contrast agent present in the field of view of the imager IA, at the exclusion of other materials. The contrast ("VC") image represents an approximation of an image that may have been obtained if there had been only contrast agent present in the field of view of the imager IA, at the exclusion of other materials. Thus, image contrast in VC is exclusively or pre-dominantly contrast agent focused. It is preferably the spectral (projection imagery) so computed by spectral processor SP that is processed by the facilitator system SYS, instead or, or in addition, to the original multi-energy input projection imagery, as will be described below in more detail.

For simplicity, the designation "$\lambda$" may be used herein to indicate input imagery in general to be processed by the facilitator system SYS. That is, "$\lambda$" may relate to original projection imagery as acquired or, preferably, to the spectral projection imagery as computed by the spectral processor SP based on the original projection imagery as acquired. The spectral processor SP and the facilitator system SYS may be implemented on different computing systems, possibly geographically separated such as in a distributed "cloud" architecture. However, implementation on a single computing system is also envisaged herein.

Imaging as envisaged herein is preferably attenuation-based, but this is not at the exclusion of other X-ray modalities, such as dark-field imaging and/or phase contrast imaging. What is more, whilst operation of system SYS on projection imagery is sufficient for most present purposes, tomosynthetic or tomographic imaging is not excluded herein, due to the multi-directional imaging capability of the preferably rotational imaging apparatus IA. A tomographic algorithm processes projection imagery from multiple directions into sectional imagery. In tomosynthetic imaging, a suitably configured algorithm adds a depth dimension to the planar projection domain. Also, use of a combination of tomographic data, possibly pre-recorded with data from the projection domain is not excluded herein. The spectral processor SP may operator in projection domain or image domain to turn the sectional imagery into spectral sectional imagery. The spectral processing algorithm may be integrated at least in parts into the reconstruction algorithm. Preferably however, operation is envisaged in projection domain without use of tomographic or tomosynthetic processing.

Input imagery $\lambda$ is processed by computer-implemented system SYS that facilitates the above-mentioned computation of one or more hemodynamic indices. One such index may include FFR or iFR. or other such index types still, such as IMR, CFR, etc., as required for the clinical task at hand. In FFR for example, pressure measurements are collected in the inflicted vessel up- and down-stream the stricture. The pressure measurements are then combined by algebraic operation. For example, a ratio based on up- and down steam pressure measurements is formed to arrive at the relevant index, for example the FFR index. This allows for example to assess the severity of the stricture. Not all strictures require per se an intervention, such as angioplasty it has been found. The index can thus inform whether or not to conduct such an interventional procedure for a given patient. The measurements may be collected by a suitable measurement device MD including suitable in-situ sensors, such as a pressure sensor, flow sensors or temperature sensors, etc. These sensors are introduced into the patient via a catheter or other, or are attached from the outside to the patient, as the case may be. However, such in-situ sensor measurements of the hemodynamic quantities are not necessarily envisaged herein in all embodiments. Instead, relevant measurements may also be taken purely image-based, based on the input imagery, spectral or not. For example, the lumen at the stricture of the inflicted blood vessel may be measured with sufficient accuracy based on projection imagery along one, or preferably, more than one projection direction. A bi-planar imaging setup with two detectors with intersecting imaging axes (for example at right angles) may be used. The imagery may be segmented for lumen, and the stricture quantified by measuring in-image geometric properties, such as length, thickness etc, of the segmentation(s). The segmentation may be well-defined thanks to the contrast provided by the contrast agent's presence and the spectral processing envisaged herein, although a conventional imaging setup, preferably with DSA, may be used instead. The bi-planar imaging setup may also be used instead for sensor-collected measurements.

As earlier mentioned, it has been found that the timing of the collection of those hemodynamic measurement, whether sensor or image-based, is of relevance. It is preferably ensured that the hemodynamic measurements are collected during one or more time periods or phases of a correct hyperemic state prevalent at or around the stricture. Hyperemia, as measured by such a state, in general refers to the amount of blood flowing, or its perfusion, at the region of interest. Hyperemia may be thought to describe an increased perfusion due to dilation of micro vessels, for example, in an autoregulatory response, to increase blood flow. Hyperemia may also directly refer to the state of least microvascular resistance.

Some such indices (eg, FFR, IMR) require that the respective measurements are collected at a (full) hyperemic state, whereas others, such as iFR, require collection at a "rest state", with a low(er) or normal perfusion that is not a hyperemic state. However, as used herein such a "rest state" may be described herein as a "low" hyperemic state. A hyperemic state may be represented by a scalar parameter h against a scale. A heightened hyperemic state indicates that there is more blood perfusion at a ROI than there is in a usual or normal, or the said rest state $H_0$. Still other hemodynamic indices such as CFR demand collection of measurements at both, high and lower hyperemic state. The hyperemic state h is thus in general a state between a maximal achievable (hyperemic) state $H^*$, or full hyperemic state, and a minimum hyperemic state $H_0$ (no hyperemia). $H^*$ thus represents the state where the maximum amount (volume/concentration) of blood perfusion is present at a given ROI, whilst $H_0$ represents the state where the normal amount of blood perfusion is present when the organism is at rest, hence "rest state". Hyperemic state $h(s)$ is in general a spatially dependent scalar as it differs in general with ROI location s for (in particular within) a given patient, although we shall drop herein in general, for the sake of simplicity, the location variable s.

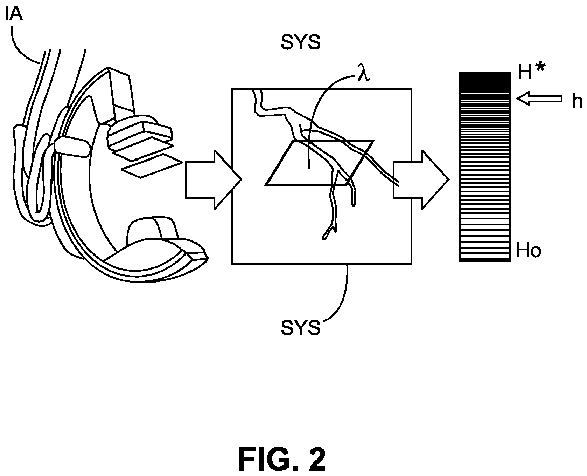

What is proposed herein is the computing system SYS as schematically shown in block diagram of Figure 2. System SYS takes as input imagery $\lambda$, and it computes therefrom the associated hyperemic state h. The parameter h so computed may be used to facilitate the collection of hemodynamic measurements at the correct hyperemic state as per h, which in turn allows computing a correct, or at least improved (eg, more accurate or more robust), hemodynamic index of the desired type. The type of hemodynamic index to be computed, and this may depend on the medical task at hand, in general determines which hemodynamic measurements need to be collected and in which state h. There is thus an association between state h and the hemodynamic measurements m. A single or more than one state may be called for when computing certain hemodynamic index types, as briefly mentioned above.

The hyperemic state h computed by system SYS may be displayed on the display device DD (or on a different display device). For example, a bar diagram may be used as shown on the right side of Figure 2, with a slider widget (or similar) thereon dynamically indicating the current hyperemic state h against the scale. For some or each frame, such a hyperemic state may be computed. Thus, the output h as provided by system SYS is preferably in real time and/or dynamic, as the hyperemic parameter h varies with the frames in the stream supplied by imager IA. Parameter h is thus in general a function of frame $f_t$, $h[f_t]$, with t a time index. A single output h for a still image is not excluded herein. It may not be necessary to compute h for each and every frame in the image steam. The computation may be skipped for some frames. The hyperemia scale may not necessarily be continuous. A binary scale may be sufficient, where the computed scalar h (after optional thresholding for example) simply indicates where or not there is hyperemia.

Operation of the system SYS may be based on administration of the contrast agent, CA although this is not necessarily required in all embodiments at all times as certain substitute substances may be used instead as will described more fully below. However, referring for now to such CA material, it has been found that such contrast agents are not only useful in improving contrast in imagery. It has been observed in addition that contrast agent itself can induce a desired hyperemic state, in particular can induce a heightened level of hyperemic state, in particular still, the full state hyperemic state $H^*$. This is because the contrast agent, as it accumulates in particular at the region of interest, displaces blood at the ROI. At one point in time, as the CA concentration increases, there is a period where there is no or very little blood present at the ROI, substantially completely displaced by the CA. This causes a physiological reaction where the body is trying to compensate for an associated momentary deprivation of oxygen as a result of the blood displacement. The compensation includes causing a vasodilation at the ROI. The blood vessel dilates, and thus admits more blood than usual to be present (after concentration of CA drops off) at the ROI, thus inducing a momentary state of heightened hyperemic state or increases hyperemia, and in particular at one point, a full hyperemia where a maximum amount of blood accumulates. What is proposed herein is the dual use by system SYS of the contrast agent, not only for enhancing of contrast in imagery, but also to control the hyperemic state so that measurements can be collected in a suitable associated hyperemic state as required by the protocol underlying the respective index.

It will be understood herein that whilst $H^*$ referrers to a maximum achievable state of hyperemia at a given ROI (and this may vary from ROI to ROI), the term "full state" of hyperemia as used herein is meant to include a merely high enough state of hyperemia, which may be less than the theoretically achievable value $H^*$. It may be sufficient in some applications to have the "full state" instead refer to a lower value, that is however higher than a certain pre-set minimum level of hyperemia. Thus, in most practical cases, $H^*$ may refer to such as minimum threshold that is needed for the collection of hemodynamic measurements for a given hyperemic index ("$h$-index") of interest. In addition, it will be understood from the above that the accumulating CA can help to induce, at one point in time, any intermediate state $h$ between $H_0$ and $H^*$. For example, an $p\%$-$h$ value may refer to a $p\%$ hyperemic state, with hyperemia at p% (eg, 75%) of $H^*$. Some $h$-indices may call for such intermediate values.

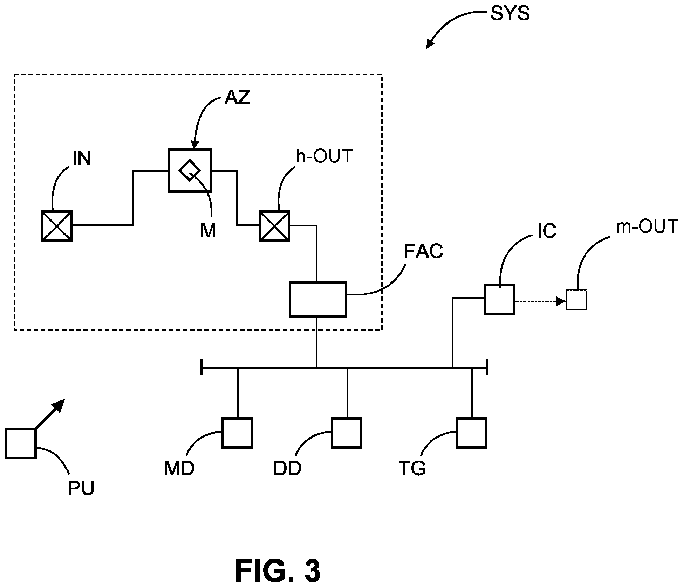

Figure 3 shows a block diagram, affording a more detailed view on the facilitator system SYS and its components. The system SYS may be implemented by one or more computing units PU, such as a desk top computer, laptop computer, smart phone or other. An implementation in the cloud, is also envisaged herein, where system SYS is implemented across one or more servers. Thus, processing may be distributed across multiple computing devices PU. Edge or cloud computing implementation, or a mix of the two, is envisaged herein. The system SYS may be implemented at least in part as "apps", an application or code, that may run on a preferably mobile computing unit PU, such as on laptop, tablet or smartphone.

At an input interface IN the input imagery λ, preferably contrasted, is received. The input λ may include a single input image or, more usual, plural imagery (such as frames of the mentioned video stream/feed) as acquired during the imaging session by imager IA. The input imagery includes preferably contrasted frames.

Online operation of the system SYS is mainly envisaged herein where the system is operable on-site, such as in the CATH-lab, during or shortly after image acquisition, although an off-line variant, operable after conclusion of the imaging session, is also envisaged. In this offline embodiment, the imagery is stored in a suitable permanent memory, and is then retrieved later, after the examination, and the system operates then on those stored images. Such storage in permanent memory may also be done in on-line application, but this is not a necessity.

An analyzer component AS of system SYS analyzes the imagery and computes, preferably per frame, a respective hyperemic state $h$. Such $h$ value may be computed for each frame, or only for some frames, such as for randomly selected frame(s), or every $k$-th>1 frame, etc. The respective state $h$ describes the state for the ROI as currently captured by the field of view of the respective frame. The region of interest may include the vessel stricture or other lesion, possibly with some neighborhood around the ROI.

The computed hyperemic state $h$ is then output via output interface h-OUT as an input signal to a facilitator component FAC. The facilitator component FAC facilitates the computation of the hemodynamic index of interest. Multiple embodiments are envisaged for the facilitator FAC either singly or in any combination as will be described below in more detail. The analyzer AN and facilitator FCA may not necessarily be implemented on the same computing unit PU, so the output interface may be internal or external.

For example, the facilitator FAC may be arranged as a control interface that controls operation of the measurement device MD such as a pressure wire/sensor, temperature sensor or flow sensor, etc, to collect measurements at suitable time points when the right hyperemic state h prevails as computed by analyzer AN.

Sensor(s) of the measurement device MD are brought into place at ROI via access point AP or other access point. A catheter may be used to position the sensor of measurement device MD at or around, for example, the stricture. Positioning of sensor(s) may be done under image-guidance using visualized imagery as acquired by imager IA.

The hyperemic state $h$ computed by analyzer AN may be used as a control signal to commence and/or cease collection of measurements. For example, if a hemodynamic index, such as FFR, is to be computed which mandates at least one collection of pressure measurement(s) in a full hyperemic state, the pressure measurement device MD is operated only when the full hyperemic state is reached, as indicated by the computed hyperemic dynamically varying parameter $h$. Thus, in this embodiment the hyperemic state $h$ is used to synchronize the collection of the hemodynamic measurements. However, such a pre-synchronization or "pre-gating" of hemodynamic measurements versus state $h$ is not necessarily required in all embodiments. For example, as an alternative or in addition, a post-gating protocol is also envisaged herein, where the measurements are collected first during a certain period regardless of the hyperemic state h. Measurement collection by measuring device MD is preferably synchronized with the frame rate irrespective of hyperemic state parameter h being computed for the respective frame. In this embodiment, the facilitator may use a tagger TG. Tagger TG tags respective frame and/the associated measurement with its respective associated hyperemic state *h.* Next, at the conclusion of measurement collection or at a later period as requested by user or automatically, a retrieval or look-up component searches among the so acquired set of measurements, to find the individual measurements that correspond to the correct hyperemic state for the index type to be computed.

For example, if an FFR index is required, the retrieval component identifies the measurements that correspond to the full hyperemic state and rest state. The measurements are thus filtered, and it is only the suitable ones that are processed by the index calculator IC to compute the respective index.

The so identified measurements at the correct hyperemic states are passed to an index computing unit IC. Index computing unit IC accesses the corresponding algebraic combiner routine or formula and combines the measurements into the desired index using the corresponding formula, eg by forming ratio, a sum, product, etc, depending on the desired index type. The computed index may be passed on to a data output interface m-OUT. For example, the computed index may be passed on to visualizer VIZ to visualize the index on display device DD in graphical, numerical, or textual form, or as combination of any two or all of the foregoing, as required. The computed index may be stored in a memory or may be otherwise processed. A natural language processing pipeline may drive an acoustic transducer to sound out the computed index.

In one embodiment, which can be used in combination with any of the above-described embodiments of facilitator FAC, the facilitator FAC merely causes display on the display device DD of an indication of the hyperemic state for the current frame. It is the user who synchronizes measurements by operating the measurement device MD when the correct hyperemic state is displayed. For example, a bar widget, horizontal or vertical as shown in Figure 2, may be displayed, optionally in conjunction with the current frame for example. In this case, the human user observes the display device DD and reads off the current hyperemic state and once the correct state is indicated, it is the operator who operates the measurement device MD at the right time to cause collection of the respective measurement at the right state *h.*

As said before, some type measurements, such a lumen diameter or other mainly geometrical properties may not be necessarily collected by in-situ sensors but can be ascertained instead purely based on the respective image frame, by segmentation for example. A given image or frame is segmented into components, such as into a vessel tree structure. The stricture location is identified, and related geometric properties of the segmented components are computed as mentioned earlier. Preferably an imaging axis is adjusted by rotation of gantry G to acquire projection images along different projection directions, in order to resolve for 3D geometric properties. Bi-plane imager may be used in alternative embodiments. Thus, in such embodiments of image-based measurements, the measuring device MD is implemented as a segmentor and image structure analyzer for measuring dimensions of the segmented components, based on projection imagery along preferably multiple different directions. Preferably the associated image axes are perpendicular such as in some bi-plane imagers, but this is not a requirement herein.

Thus, as descried above the computed hyperemic state *h* may be used for hemodynamic measurement versus hyperemic state association. Such measurement versus hyperemic state association may include controlling measurement device and/or synchronization in post- or pre-gating protocols (as described above). Instead of, or in addition to such measurement versus hyperemic state association, the computed hyperemic state *h* may be used herein for other control tasks, such as any one or more of: controlling of the contrast delivery device DA, such as stopping of injection, start of injection of CA or another substance other than CA such as saline solution (on which more further below), or performing some imaging control actions, such as de-activating the X-ray source XS, such as switching source off, once the examination concluded , etc, and/or other such imaging support tasks.

The system SYS may implement suitable, preferably wireless, communication protocols, such as Wi-Fi (IEEE 802.11), Bluetooth®, NFC, etc, for receiving imagery $f_t$ and/or hemodynamic measurements $m_t$, in particular in online, real-time applications, preferably (but not only) when implemented at least in parts by a mobile computing unit PU. The in-situ sensors, if any, may be communicatively coupled to suitable transmitters for wireless transmission of measurements $m_t$ to system SYS for processing. Whilst wireless communication protocols are envisaged, wired solutions are not excluded herein.

As mentioned above, modulation of the hyperemic state is partly influenced and induced by the presence of the contrast agent which influences (eg, displaces) the amount of blood present at the respective region of interest. As the concentration of the contrast agent is varying, so is the hyperemic state *h.* In a broad and rough approximation, a suitable degree of correlation may be assumed between the concentration or amount of contrast agent at the region of interest with the hyperemic state. This modelling assumption can be leveraged herein by mapping the concentration of the contrast agent to an associated hyperemic state *h.* Thus, analyzer AN may be implemented in some embodiment as a look-up table or other analytic set-up that describes this correlation as an analytic function. Prior tests may be conducted using prior data (such as spectral image data) and expert knowledge to construct such a function. An explicit computation of the CA concentration/amount itself may not be required. The analyzer AN preferably processes input imagery (preferably spectral, but this is not a necessity), and maps onto hyperemic state value *h.*

However, the above mentioned analytical correlation is a rough approximation. It has been found that more complex physiological effects are at play. As mentioned above, for example the displacement of blood for a certain period of time by the contrast agent is causing a kind of "fight and flight" reaction of the body where vasodilation occurs, thus leading to the heightened hyperemic state. However, the precise onset of the vasodilation may differ from patient to patient due to their physiological characteristics such as body mass index, age, sex, patient history etc. Also, it has been observed that the CA-induced vasodilation may not necessarily set in exactly when the maximum amount of contrast agent has accumulated. Instead, there may be a certain delay $\Delta$ after maximum CA-concentration, after which the vessel dilates, and possibly another delay $\Delta'$ during which blood is beginning to fill this dilated section of the vessel. Modelling the applicable physiological characteristics and/or the mentioned delay factors $\Delta$, $\Delta'$ analytically may be challenging. Thus, in order to better account for these physiological effects and/or delays $\Delta$, $\Delta'$, in a preferred embodiment the analyzer AN uses machine learning (ML).

In ML approaches, this relationship between image-observable contrast agent concentration/volume and hyperemic state value $h$ is learned implicitly from training data, and does not necessarily require an explicit analytic ad hoc modelling. Instead, a generic machine learning model $M$ architecture may be used instead. Based on previous training data, as may be found in medical data bases, this relationship is implicitly modelled by adjusting certain parameters of the machine learning model based on the training set in a training phase. Once sufficiently trained (as may be determined by tests), the trained model M can then be deployed into medical practice as described above to estimate the hyperemic state accurately and robustly.

Additional learning input may be provided to enrich the image input $\lambda$ by contextual data c. The contextual data $c$ may include some of all of the physiological patent characteristic, and/or some of the in-situ measurements. The contextual data may include pre-operative 3D imagery, such as CT, MRI or other, so that the model M can take surrounding anatomy into account for better learning.

Initial (for example arbitrarily chosen) parameters $\theta^k$ of the machine learning model M can be adapted in on or more iterations $k$, based on the training data (possibly enriched by contextual data c) to capture this latent mapping or relationship between $h$ and contrast agent concentration/amount. However, as mentioned above, such a machine learning model approach is not necessarily required and other, in particular analytical modelling approaches are also envisaged as they may provide results in sufficient approximation in some instances.

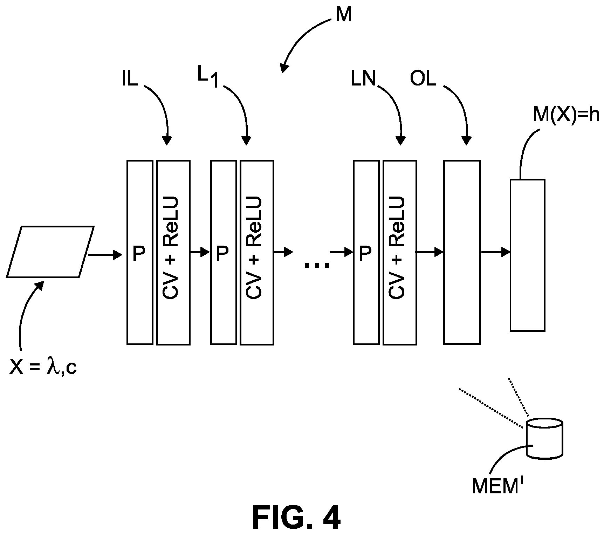

Referring now to Figure 4 this shows a block diagram of a machine learning model M as may be used herein in some embodiments. The machine learning model may be arranged as an artificial neural network ("NN"), preferably as an NN of the convolutional type, referred to as "CNN". CNNs are useful in processing spatially correlated data such as image data, the processing of which is envisaged herein as explained earlier.

The network M may comprise a plurality of computational nodes arranged in layers in a cascaded fashion, with data flow proceeding from left to right and thus from layer to layer. The model may thus be of the feedforward type. Recurrent networks are not excluded herein.

The model network M may be said to have a deep architecture because it has more than one hidden layers. In a feed-forward network, the "depth" is the number of hidden layers Lj ($1 \leq j \leq N$) between input layer IL and output layer OL, whilst in recurrent networks the depth is the number of hidden layers, times the number of passes.

The machine learning model includes certain parameters, such as weights of convolution filters CV, that are adjusted in the training phase which will be explained in more detail below. Once trained, that is, once the parameters are suitably adjusted, input X, including input contrasted imagery $\lambda$ (in particular contrasted projection imagery $\lambda$), possibly enriched by contextual data c, is input and received at an input layer IL during deployment or testing. The input X is hence either $\lambda$ or ($\lambda$, c). The input data X in deployment or testing propagates through the layers of the model and emerges as the desired result M(X)=h as the output layer OL. Thus, at output layer OL, the desired result is produced including in some embodiments the hyperemic state h. Thus the output may be a regressed into a scalar value $h$ preferably one from a bounded interval [$a$,$b$], for example $a$ = "$0$" and $b$ = "$1$" indicating the respective level of hyperemia. For example, $a$="$0$" may represent a rest state $H_0$, whilst "$1$" indicates full hyperemic state $H^*$. However, this is a matter of coding and any other coding or mapping to other numbers is also envisaged herein, as required. Instead of regression, a classification may be sufficient in some embodiments.

Thus, the machine learning model M, for example the neural network example shown in Figure 4 or others, can be understood to act as a transformer, where the two or higher dimensional data ($\lambda$ or ($\lambda$,c) ) is transformed into a single scalar value h, as the input data progresses through the layers IL, $L_j$ ($j \geq 1$),OL of the network $M$.

The output layer OL may be a regression layer where the input data, including imagery $\lambda$ or ($\lambda$,c) is regressed into a single scalar value $h$. Alternatively, a classifier layer OL may be used, where the input is classified into $h$-value-levels "rest", full, or "intermediary" (generic or atp%), as such qualitative output may be sufficient to control $h$ versus measurement $m$ association in some cases. In this case output may be a vector with its entries representing the classes of $h$-value levels. A single level may be sufficient in some instance, where the model is trained into a binary classifier to classify into for example, *"rest state, yes/no?"* or *"full state, yes/no?"*, etc.

In case of a classification result, the output layer OL may be configured as a *softmax*-function layer or as similar computational nodes where feature maps from previous layer(s) are combined into normalized counts to represent the classification probability or scores per class. In a regressional setup, OL may be fully connected layer.

Preferably, some or all of the hidden layers Lj ($1 \leq j \leq N$), and optionally the input layer, are convolutional layers, that is, include one or more convolutional filters CV which process an input feature map from an earlier layer into layer-output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, *tanh*-function or any other suitable non-linear function. As opposed to a convolution layer, the output layer may be fully connected layer. Hybrid networks, including fully connected and convolutional layers are also envisaged herein.

Additional optional layers may be used in CNN model M, such as drop-out layer, pooling layers P, and other. The pooling layers reduce dimension of output whilst drop-out layer sever connections between nodes from different layers.

A hybrid model is also envisaged, optionally including a pre-processing network for example, such as an autoencoder (AE) or a variational AE (VAE). The preprocessor preprocesses the input first to produce intermediate output and it is this intermediate output that is then classified or regressed into the hyperemic state $h$ as described above. For example, the preprocessor AE may be trained to segment the input image for main vessels, micro vessels and perfused tissue for example. Thus, portions of the input image may be so classified first into segmentation classes. Then, for each class, the remaining network may determine contrast amount in each vessel class/ hyperemic state. Thus, in general, the preprocessor of whichever kind, AE or not, may be used to produce location dependent h(s) values as opposed to a global value for the whole image. Both, localized or global h value computations are envisaged herein in embodiments.

The trained model may be stored in one or more data storages MEM'.

Preferably, to achieve good throughput, the computing device PU includes one or more processors (CPU) that support parallel computing, such as those of multi-core design. In one embodiment, GPU(s) (graphical processing units) are used.

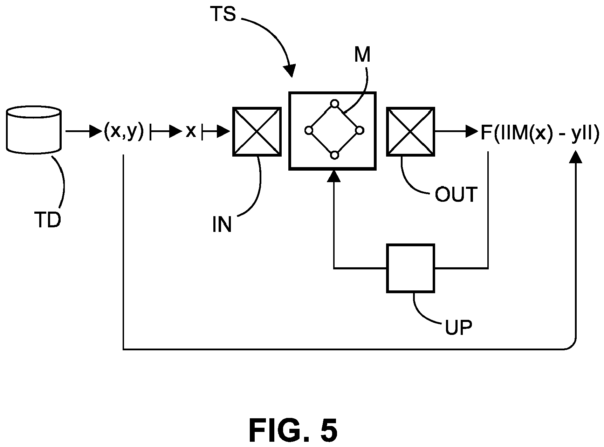

Referring now to Figure 5, this shows a training system TS that may be used to train such a model M based on training data $(x,y)$ as may be held in a training data memory TD. Training data $(x,y)$ can be procured from existing medical data such as may be found in a medical data bases, such a PACS, HIS or other database or storage system. Such training data may include historic image data or other health records of patients that have undergone similar examinations in which hemodynamic indices were computed, or at least where contrasted imagery of a related ROI was acquired. The historic imagery may be reviewed by a human expert, for example. The expert may review the contrasted imagery of historic patients and may select historic imagery that corresponds, according to their medical knowledge, to a certain hyperemic state. The human expert may review the patient health record for notes on the respective patient to come to their conclusion. Thus, the human expert may assign a respective score $y$ (or label) to different respective samples of training input imagery x. Score $y \in [a.b]$, on a predefined scale $[a,b]$. Score $y$ represents an estimate of the respective hyperemic state associated by the expert with the respective input image $X$.

Respective (historic) context data c, such as historic hemodynamic measurement, etc may be used to assist the user when assigning their score y. The context c may be found in the health record of historic patients. In some cases, the health record context $c$ may be used to infer the correct hyperemic state $h$. Specifically, medical records associated with historic imagery may be harvested either by a human user or by an automatic text analyzer tool (such as an NLP pipeline or string matcher tool, such as grep or other) to extract the corresponding hyperemic state as this information may be sometimes mentioned in the respective reports or records referring to a specific image frame. In this way, the collection process can be fully or partially automated, thus allowing to procure suitably labelled training data construct more quickly.

Training as administered by training system TS is the process of adapting parameters of the model based on the training data. An explicit model is not necessarily required as in some examples it is the training data itself that constitutes the model, such as in clustering techniques or k-nearest neighbors. etc. In explicit modelling, such as in NN-based approaches and many others, the model may include as system of model functions/computational nodes, with their input and/or output it least partially interconnected. The model functions or nodes are associated with parameters θ which are adapted in training. The model functions may include the convolution operators and/or weights of the non-linear units such as a RELU, mentioned above at Figure 4 in connection with NN-type models. In the NN-case, the parameters θ may include the weights of convolution kernels of operators CV and/or of the non-linear units.

The parameterized model may be formally written as $M^\theta$. The parameter adaptation may be implemented by a numerical optimization procedure. The optimization may be iterative. An objective function f may be used to guide or control the optimization procedure. The parameters are adapted or updated by updater UP so that the objective function is improved. The input training data $x^i$ is applied to the model. The model responds to produce training data output $M(x^i) = y^i$. The objective function is mapping from parameters space into a set of numbers. The objective function $f$ measures a combined deviation between the training data outputs $y^1$ and the respective targets $y^1$. Parameters are iteratively adjusted to that the combined deviation decreases until a user or designer pre-set stopping condition is satisfied. The objective function may use a distance measure $\| \, . \, \|$ to quantify the deviation.

In some embodiments, but not all, the combined deviation may be implemented as a sum over some or all residues based on the training data instances/pairs $(x^i, y^i)^i$, and the optimization problem in terms of the objective function may be formulated as:

$$argmin_\theta f = \sum_k || M^\theta(x_k), y_k || \qquad (1)$$

In the setup (1), the optimization is formulated as a minimization of a cost function $f$, but is not limiting herein, as the dual formulation of maximizing a utility function may be used instead. Summation is over training data instances $i$.

The cost function $f$ may be pixel/voxel-based, such as the L1 or L2-norm cost function. For example in least squares or similar approaches, the (squared) Euclidean-distance-type cost function in (1) may be used for the above mentioned regression task. When configuring the model as a classifier, the summation in (1) is formulated instead as one of cross-entropy or Kullback-Leiber divergence or similar. Huber cost function may be used.

The exact functional composition of the updater UP depends on the optimization procedure implemented. For example. an optimization scheme such as backward/forward propagation or other gradient based methods may be used to adapt the parameters $\theta$ of the model M so as to decrease the combined residue for all or a subset $(x_k, y_k)$ of training pairs from the full training data set. Such subsets are sometime referred to as batches, and the optimization may proceed batchwise until all of the training data set is exhausted, or until a predefined number of training data instances have been processed.

Training may be a one-off operation, or may be repeated once new training data become available.

Optionally, one or more batch normalization operators ("BN", not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

The training system as shown in Figure 5 can be considered for all learning schemes, in particular supervised schemes. Unsupervised learning schemes may also be envisaged herein in alternative embodiments. GPUs may be used to implement the training system TS.

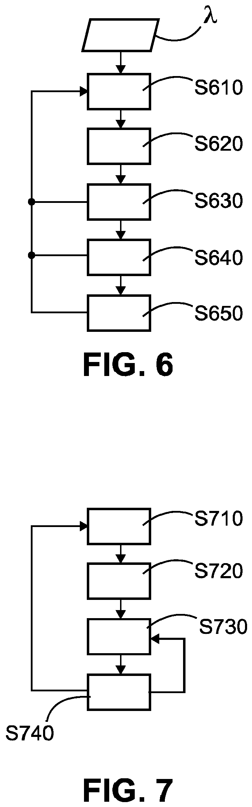

Reference is now made to Figure 6 which shows a flow chart of a method of facilitating computation of a hemodynamic index or such indices. The method may be used to implement the above system SYS although it will be understood that the below described method may also be used in its own right, without being necessarily tied to the system architecture defined above.

At step S610 the one or more input imagery is received of a region of interest. The input imagery includes or is based on contrasted imagery as described above. Imagery may further include non-contrasted imagery, especially if DSA is to be used. The imagery is preferably spectral imagery computed from contrasted imagery. Projection imagery is preferably used, but reconstructed tomographic or tomosynthetic imagery is not excluded herein, spectral or not.

At step S620 the imagery is analyzed to compute a hyperemic state. The hyperemic state is computed preferably per frame. The computation may be optionally based on context data, such as acquired hemodynamic measurements in relation to the ROI.

At step S630 a signal related to the computed hyperemic state value h is output. For example, the state $h$ may be output. The output $h$ may be made available for facilitating at step S640 the computation of a hemodynamic index of the desired type, or of plural such indices.

For example, the facilitation may include associating hemodynamic measurements with their respective patient state h. This may be written formally as $m(k) \leftrightarrow h(k)$, wherein "$k$" is a time instant that indicates collection of measurement at time $k$, and $k$ further relates to the acquisition time of frame $f[k]$, based on which frame $f$ the hyperemic state $h$ was computed. For example, the facilitation may include pre-gating or post-gating measurements $m$ of hemodynamic quantities by suitable sensors (pressure flow, temperature, etc) as described above. In pre-gating, measurements are taken only at instants when the computed value h indicates the correct pre-defined state for a given frame. In post-gating, measurements are taken over a period regardless the associated value $h$. The measurements are however tagged with their $h$ value. In a subsequent processing step, all measurements associated with the correct value $h$ are retrieved or identified based on the tag.

The step S640 of facilitating may include computing the hemodynamic index based on hemodynamic measurements associated with the correct hyperemic state as computed at step S630. The correct hyperemic state is predetermined by the respective type of hemodynamic index one wishes to compute.

For example, in embodiment, the hemodynamic index to be computed is specified by the user or is automatically selected based on protocol. Based on the selection of the hemodynamic index to be computed, its associated hyperemic state of plural such (different) states are identified. This can be done in a simple look-up operation as the correct state or group of states is a priori known. Once the correct hyperemic state(s) is known, the hemodynamic measurements associated with the correct states are identified. It is the so identified hemodynamic measurements that are then combined in a combination sub-step of step S640 to arrive at the hemodynamic index. For example, the combination sub-step may include algebraically processing the identified measurements by taking averages, computing coefficients, computing ratios, etc. Because the computation is based only on measurements at the correct hyperemic state, an improved accuracy of the indices can be ensured.

The output *h* may be used for controlling a display device. An indication of the hyperemic state *h* may be displayed, such as by a number, graphical symbology (bar chart, etc) that changes color and/or hue, position of a widget, etc, as required. Instead of operating a display device DD, a simple flashlight or lamp may be activated in a patten to indicate the current state h. Transducers other than visual can be controlled instead of or in addition, such as an acoustic transducer to sound out an audio signal which encodes the current state *h.*

In an optional embodiment the information on the over-time hyperemic state evolution as computed per frames may be stored. The so recorded h-value-evolution data can be used for substances other than for contrast agents, that are known to have similar hyperemic state inducing properties. For example, saline solutions are often times administered to the patient PAT during such an intervention for flushing for example. Thus, in this, "surrogate"-embodiment, the hyperemic state evolution as caused previously by the contrast agent, is now used instead for such a non-contrast agent substance administered to the patient.

Specifically in this embodiment at an optional step S650, based on the time instants of when the desired hyperemic state was computed for the contrast agent as per the *h*-evolution data, associated elapsed periods of time after CA administration are recorded. Then, after administration of the non-contrast substance to the patient, the so identified time periods are allowed to elapse and are used to control association of a new set of measurements, by gating or port-gating as required, with their respective hyperemic state. Thus, a second set of another or the same index/indices may be computed if required. In other words, this allows the user to compute more indices at the correct hyperemic states on different occasions even after the contrast agent has been administered.

Contrast agent may have adverse health effects on the patient. So it is not advisable to keep administering them solely for the purpose of measuring the hemodynamic indices. Thus, if the protocol envisages administration of a harmless substance that is nevertheless known to induce hyperemia similar to the CA, this effect can be harnessed herein as described above to facilitate obtaining further set(s) of such hemodynamic indices.

In fact, saline solutions or similar solutions are in general harmless to most patients and can in fact be repeatedly administered once the time evolution of the hyperemic state h has been learned in an initial phase during contrast agent administration as described above.

Step S640 of facilitating may not only include controlling measurement collection in a pre-gating or post-gating fashion, but may also include, instead or in addition, displaying of the indication of the computed hyperemic state on the display device so that the user can commence at the appropriate time with the collection of the measurements.

As mentioned above, the measurements may be based on sensors such as pressure, flow, temperature or otherwise. Alternatively, measurements may be purely or at least partly image based such as computing geometric or other properties of segmentation(s).

The analyzing step SS620 may be done based on the imagery as such, or may be based in addition or instead on CA information extracted from the imagery. The analyzing operation S620 may thus include such an extraction step where CA measures are extracted. Measures that are extracted are: total Iodine dose, injection length, blood Iodine concentration in the vessels, Iodine concentration in the tissue. Iodine is merely an example of a contrast agent envisaged. Other CAs may be used instead.

In some embodiments, the hyperemic state *h* is derived from the CA (such as Iodine) measures so extracted. For example, from a blood Iodine mixing ratio and the injection length it may be determined if full hyperemia is reached. For example, concentration and injection length should preferably be both above predefined thresholds to reliably conclude that full hyperemia is present. Alternatively or in addition, from remaining iodine concentration in the tissue, a residual hyperemic state (a non-resting/intermediate state) may be detected. The reduction of tissue iodine concentration over time allows to estimate the patient specific time it takes for the hyperemic state to fully subside into the resting state. "Injection length" as referred to above includes volume of the CA administered and/or injection speed. Mixing ratio/volume defines the percentage of deficient oxygenation, whereas injection length defines for how long there is deficient oxygenation.

As an alternative to non-ML approaches as described based on CA measures, ML may be used. ML is based on training data, as described above at Figs 4,5. The training data preferably incudes spectral imagery, such as dual energy X-ray data, and hemodynamic data (e.g. pressure curves). This data can be used to train an ML model M to correlate hyperemic state to CA measures, or directly CA to spectral data, such as dual-layer data, photon-counting data, or other. In ML approaches, the CA measure extraction operations may be omitted, but may be helpful in non-ML approaches. However, such CA measure extraction may still be combined with ML setup of required. ML allows computing hyperemic states end-to-end without prior CA measure calculations/extractions, etc.

As another application of the computed hyperemic state, correction of a measured value or of given a diagnostic index is envisaged, in case the measurement was erroneously taken during partial/intermediate hyperemia for example, even though the definition of the relevant diagnostic index requires full hyperemia. In this case, the computation can be redone by using the measurements associated with the correct h-value. Thus, the facilitation at step S640 envisaged herein includes such correction operation of previously calculated indices albeit based on wrong states.

The hemodynamic measurement as acquired in the facilitating step may use the same angiography data as for the CA measure extraction, or may be performed by the mentioned measurement device MD, such as by flow or pressure wires, or temperature sensor. If an in-situ measurement device is used, it is preferable to ensure a temporal correlation between the hemodynamic measurement collection and the angiography or other image data. The frame rate may be used to trigger measurements for example.

Hyperemia state estimation in step S620 may further be supported by invasive pressure or flow data. E.g. a peak pressure gradient is used to identify peak hyperemia. This allows to calibrate the contrast-based estimation for possible patient specific delays in the microvascular response. Another example is to compare peak gradients from pressure data for different CA injections. If a small contrast dose leads to the same pressure gradient as a large dose, this may be taken as an indication that the resting state is close to the hyperemic state.

Reference is now made to the flow chart in Figure 7 which shows a training method of training a machine learning model M as may be used herein in embodiments.

At step S710 training data is received, for example in the form of pairs $(x_k, y_k)$. Each pair includes the training input $x_k$ and the associated target $y_k$. $x_k$, as defined above.

At step S720, the training input $x_k$ is applied to an initialized machine learning model M to produce a training output.

A deviation, or residue, of the training output $M(x_k)$ from the associated target $y_k$ is quantified by a cost function $F$ at step S730. One or more parameters of the model are adapted at step S740 in one or more iterations in an inner loop to improve the cost function. For instance, the model parameters are adapted to decrease residues as measured by the cost function. The parameters include in particular weights of the convolutional operators, in case a convolutional model M is used.

The training method then returns in an outer loop to step S710 where the next pair of training data is fed in. In step S740, the parameters of the model are adapted so that the aggregated residues of all pairs considered are decreased, in particular minimized. The cost function quantifies the aggregated residues. Forward- backward propagation or similar gradient-based techniques may be used in the inner loop. Instead of looping over individual pairs at step S710, looping is over stets of training data pair, or batches, and these are feed in a processed at once in step S720, and S730.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

More generally, the parameters of the model M are adjusted to improve objective function F which is either a cost function or a utility function. In embodiments, the cost function is configured to the measure the aggregated residues. In embodiments the aggregation of residues is implemented by summation over all or some residues for all pairs considered. In particular, in preferred embodiments the outer summation proceeds in batches (subset of training instances), whose summed residues are considered all at once when adjusting the parameters in the inner loop. The outer loop then proceeds to the next batch and so for until the requisite number of training data instances have been processed.

In any of the above described embodiments, hemodynamic measurements envisaged herein includes thermodilution.

The components of system SYS may be implemented as one or more software modules, run on one or more general-purpose processing units PU such as a workstation associated with the imager IA, or on a server computer associated with a group of imagers. The workstation or other data processing unit(s) PU may be part of the imaging apparatus IA.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above. According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. System (SYS) for image processing, comprising:

   an input interface (IN) for receiving input data including projection imagery of a patient with contrast agent acquired by an X-ray based imaging apparatus (IA);
   an analyzer (AZ) configured to establish a patient state, including a hyperemic state or a resting state, based on the projection imagery; and
   an output interface (h-OUT) for providing an output signal indicative of the established state for facilitating obtaining a hemodynamic index in relation to the patient.

2. System of claim 1, including a facilitator (FAC) operative to associate a respective hemodynamic measurement with a respective patient state to obtain associated data.

3. System of claim 1 or 2, wherein the system comprises an index computation component (IC), operable to compute one or more hemodynamic indices based on the associated data.

4. System of any one of claims 2-3, wherein the facilitator (FAC) is configured to facilitate collection of hemodynamic measurements based on the established patient state.

5. System of any one of the previous claims, wherein the analyzer (AZ) is based on a trained machine learning model (M).

6. System of any one of the previous claims, wherein the facilitator (FAC) includes a visualizer (VIZ) configured to effect display on a display device (DD) of an indication of the established state.

7. System of any one of the previous claims 3-6, a measurement output data interface (m-OUT) for providing one or more such hemodynamic measurements.

8. System of any one of the previous claims, the analyzer (AZ) to establish an over- time behavior of the patient state, the analyzer (AZ) to use the same over-time behavior for establishing a next patient state of the patient, with a non-contrast agent substance instead of the contrast agent, the non-contrast agent substance capable of inducing a patient state similar to the patient state induced by the contrast agent.

9. System of any one of the previous claims, wherein the imaging apparatus (IA) is configured for spectral imaging.

10. System of any one of the previous claims, wherein the imaged region of interest includes at least a part of the heart of the patient or a peripheral vessel.

11. A medical imaging arrangement (MIA), including the system of any one of the previous claims and any one of more of i) the imaging apparatus (IA) ii) the display device (DD), iii) a contrast or non-contrast agent delivery apparatus (DA), iv) device (MD) configured (PW) to acquire the hemodynamic measurements.

12. The arrangement of claim 11, further including any one of more of: i) the display device (DD), ii) a contrast or non-contrast agent delivery apparatus (DA), iii) device (MD) configured (PW) to acquire the hemodynamic measurements.

13. Computer-implemented method for image-processing, comprising:

   receiving (S610) input data including projection imagery of a patient with contrast agent acquired by an X-ray based imaging apparatus (IA);
   establishing (S620) a patient state, including a hyperemic state or a resting state, based on the projection imagery; and
   providing (S630) an output signal indicative of the established state for facilitating obtaining a hemodynamic index in relation to the patient.

14. A computer program element, which, when being executed by at least one processing unit, is adapted to cause the at least one processing unit (PU) to perform the method as per claim 13.

15. At least one computer readable medium (MEM) having stored thereon the program element of claim 14, or having stored thereon the trained machine learning model as per claim 5.

**FIG. 1**

**FIG. 2**

**FIG. 3**

**FIG. 4**

**FIG. 5**

λ

S610

S620

S630

S640

S650

# FIG. 6

S710

S720

S730

S740

# FIG. 7

Europäisches
Patentamt

European
Patent Office

Office européen
des brevets

# EUROPEAN SEARCH REPORT

Application Number

EP 22 17 3972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/042569 A1 (SAKAGUCHI TAKUYA [JP] ET AL) 15 February 2018 (2018-02-15) * paragraphs [0036], [0039] * ----- | 1-15 | INV. A61B6/00 A61B8/08 |
| A | JP 6 362851 B2 (TOSHIBA CORP; TOSHIBA MEDICAL SYS CORP) 25 July 2018 (2018-07-25) * paragraph [0048] * ----- | 1-15 | |

TECHNICAL FIELDS
SEARCHED      (IPC)

A61B

The present search report has been drawn up for all claims

| Place of search | Date of completion of the search | Examiner |
|---|---|---|
| Munich | 20 October 2022 | Anscombe, Marcel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

**EP 22 17 3972**

This annex lists the patent family members relating to the patent documents cited in the above-mentioned European search report.
The members are as contained in the European Patent Office EDP file on
The European Patent Office is in no way liable for these particulars which are merely given for the purpose of information.

**20-10-2022**

| Patent document cited in search report | | Publication date | Patent family member(s) | | Publication date |
|---|---|---|---|---|---|
| US 2018042569 | A1 | 15-02-2018 | JP | 6918629 B2 | 11-08-2021 |
| | | | JP | 2018027307 A | 22-02-2018 |
| | | | US | 2018042569 A1 | 15-02-2018 |
| | | | US | 2020214659 A1 | 09-07-2020 |
| JP 6362851 | B2 | 25-07-2018 | JP | 6362851 B2 | 25-07-2018 |
| | | | JP | 2015097724 A | 28-05-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2, , 6